# EUROPÄISCHE PATENTSCHRIFT

(11) **EP 0 939 610 B1**
(45) Veröffentlichungstag und Bekanntmachung des Hinweises auf die Patenterteilung: **03.09.2003**
(21) Anmeldenummer: 97948877.2
(22) Anmeldetag: 04.11.1997
(51) Int. Cl.: A61K 7/06, A61K 7/48, A61K 7/50

(54) **KOSMETISCHE ZUBEREITUNGEN**
COSMETIC PREPARATIONS
PREPARATIONS COSMETIQUES

(30) Priorität: 13.11.1996 DE 19646867
(43) Veröffentlichungstag der Anmeldung: 08.09.1999
(73) Patentinhaber: Cognis Deutschland GmbH & Co. KG, 40589 Düsseldorf (DE)
(72) Erfinder: ANSMANN, Achim, D-40699 Erkrath (DE); FABRY, Bernd, D-41352 Korschenbroich (DE)
(86) Internationale Anmeldenummer: EP9706085
(87) Internationale Veröffentlichungsnummer: WO98020845

(56) Entgegenhaltungen:
- EP-A- 0 264 844
- EP-A- 0 535 367
- WO-A-94/16677
- WO-A-98/03155
- DE-A- 4 243 272
- FR-A- 2 134 451
- DATABASE WPI Section Ch, Week 7418 Derwent Publications Ltd., London, GB; Class D21, AN 74-33197V XP002060739 & JP 48 033 037 A (POLA CHEM IND CO) , 7.Mai 1973

## Beschreibung

### Gebiet der Erfindung

Gegenstand der Erfindung sind kosmetische Zubereitungen, enthaltend (a) ausgewählte Periglanzwachse, (b) Siliconverbindungen und (c) ausgewählte Emulgatoren sowie die Verwendung der Mischungen zur Herstellung von perlglänzenden Siliconshampoos.

### Stand der Technik

Noch vor 15 Jahren setzten sich Haarshampoos in der Regel nur aus Wasser und Tensiden zusammen, was vom Gesichtspunkt der Reinigung und Entfettung des Haares zweifellos vollkommen ausreichend war. Mit der Aufklärung des Verbrauchers über Risiken im Umgang mit kosmetischen Inhaltsstoffen und dem Wunsch nach Produkten, die nicht nur reinigen, sondern auch pflegen, sind die Anforderungen an moderne kosmetische Zubereitungen im allgemeinen und Haarshampoos im besonderen ständig gestiegen. Der Verbraucher erwartet völlig zu Recht, daß die Produkte ein Maximum an hautkosmetischer Verträglichkeit aufweisen, d.h. daß auch bei häufigem Gebrauch durch besonders empfindliche Personen Irritationen der Haut und der Schleimhaut zuverlässig unterbleiben. Im Bereich der Pflege wird erwartet, daß die Zubereitungen auch die Kämmbarkeit der Haare verbessern, also avivierend, konditionierend und antistatisch wirken. Schließlich ist erwiesen, daß die Ästhetik der Zubereitungen, also beispielsweise ein brillanter Perlglanz, das Kaufinteresse des Verbrauchers positiv beeinflussen.

Moderne Haarshampoos des Stands der Technik enthalten aus den genannten Gründen häufig milde Tenside, Perlglanzwachse wie beispielsweise Ethylenglycolbisstearat und Siliconverbindungen mit avivierenden Eigenschaften. Die Formulierungsmöglichkeiten dieser Produkte sind jedoch begrenzt, da Silicone die Neigung zeigen, in wäßrigen Lösungen auszutrüben und eine organische Phase zu bilden, was dazu führt, daß das optische Erscheinungsbild des Produktes leidet und der Perlglanz an Brillanz verliert. Eine Übersicht zu modernen perlglänzenden Formulierungen findet sich von A.Ansmann et al. in **Parf.Kosm. 75, 578 (1994).**

Perlglanzkonzentrate, die acylierte Ethylenglycole als Perlglanzwachse zusammen mit Alkylglucosiden enthalten, sind beispielsweise aus den beiden Europäischen Patentschriften **EP-B1 0376083** und **EP-B1 0570398** (Henkel) bekannt. Zusammensetzungen, die Alkyloligoglykoside, bei Raumtemperatur flüssige Silicone und Perlglanzwachse vom Typ der acylierten Ethylenglycole enthalten, beschreiben die beiden Europäischen Patentschriften **EP-B1 0377354** und **EP-B1 0398177** (Kao).
Haarwaschmittel, die Siliconverbindungen, Detergenzien und Dialkylether enthalten, sind aus der **WO 98/03155** bekannt.

Demzufolge hat die komplexe Aufgabe der Erfindung darin bestanden, neue kosmetische Zubereitungen, insbesondere perlglänzende Siliconshampoos, zur Verfügung zu stellen, die sich gleichzeitig durch einen brillanten Perlglanz, hohe Lagerstabilität, ausgezeichnete konditionierende und avivierende Eigenschaften und besondere hautkosmetische Verträglichkeit auszeichnen.

### Beschreibung der Erfindung

Gegenstand der Erfindung sind kosmetische Zubereitungen, enthaltend
(a) Dialkylether der Formel **(I),**

   **R**^{**1**}**-O-R**^{**2**} **(I)**

   in der R¹ und R² unabhängig voneinander für lineare oder verzweigte Alkyl- und/oder Alkenylreste mit 12 bis 22, vorzugsweise 16 bis 18 Kohlenstoffatomen stehen,
(b) Siliconverbindungen und
(c) Emulgatoren ausgewählt aus der Gruppe, die gebildet wird von Alkyl- und/oder Alkenyloligoglykosiden und/oder Fettsäure-N-alkylpolyhydroxyalkylamiden, wie definiert im Patentanspruch 1.

Überraschenderweise wurde gefunden, daß durch geeignete Auswahl von Perlglanzwachsen und Emulgatoren Mischungen erhalten werden, die in der Lage sind, Siliconverbindungen in wäßrigen Zubereitungen, insbesondere Haarshampoos, zu stabilisieren, so daß die Produkte die erforderliche Lagerstabilität aufweisen. Gleichzeitig stellt man beim Zusammenwirken der Distearylether und der Siliconverbindungen eine synergistische Verbesserung sowohl des Perlglanzes als auch der avivie-renden und konditionierenden Eigenschaften fest. Die Erfindung schließt die Erkenntnis ein, daß die Mischungen zudem eine besonders vorteilhafte hautkosmetische Verträglichkeit aufweisen.

### Dialkylether

Dialkylether, die als perlglänzende Komponente (a) in Betracht kommen, werden üblicherweise durch Kondensation entsprechender Fettalkohole herstellt **[Bull.Soc.Chim.France 333 (1949)**]. Typische Beispiele sind Dilaurylether, Dimyristylether, Dicetylether, Diisostearylether, Dioleylether, Dibehenylether und Dierucylether, vorzugsweise wird Distearylether eingesetzt. Die Perlglanzwachse können eine durchschnittliche Teilchengröße im Bereich von 0,1 bis 20, vorzugsweise 5 bis 15 und insbesondere 12 bis 14 µm aufweisen.

### Siliconverbindungen

Geeignete Siliconverbindungen, die als Komponente (b) in Betracht kommen, sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconen, die bei Raumtemperatur sowohl flüssig als auch harzförmig vorliegen können.

### Alkyl- und/oder Alkenyloligoglykoside

Alkyl- und Alkenyloligoglykoside, die als Emulgatorkomponente (c) in Betracht kommen, stellen bekannte nichtionische Tenside dar, die der Formel (II) folgen,

**R**^{**3**}**O-[G]**_{**p**} **(II)**

in der R³ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie, beispielsweise durch sauer katalysierte Acetalisierung von Glucose mit Fettalkoholen erhalten werden.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(II)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R³ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R³ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren tech-nische Gemische, die wie oben beschrieben erhalten werden können. Bevorzugt sind Alkyloligogluco-side auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide, die ebenfalls als Emulgatorkomponente (c) in Betracht kommen, stellen nichtionische Tenside dar, die der Formel **(III)** folgen, 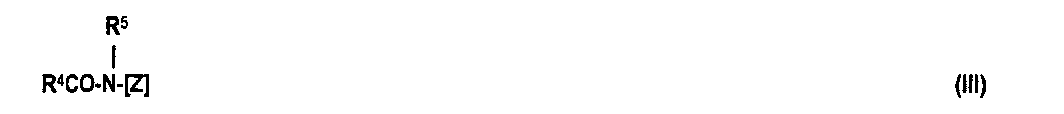 in der R⁴CO für einen aliphatischen Acylrest mit 6 bis 22 Kohlenstoffatomen, R⁵ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens.Surf. Deterg. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(IV)** wiedergegeben werden:

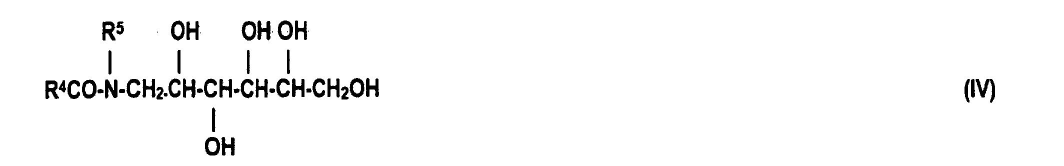

Vorzugsweise werden als Fettsäure-N-alkylpolyhydroxyalkylamide Glucamide der Formel (IV) eingesetzt, in der R⁵ für eine Alkylgruppe steht und R⁴CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(IV)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Kosmetische Zubereitungen

Der Anteil der Komponenten (a), (b) und (c) an den erfindungsgemäßen Zubereitungen kann - bezogen auf den Feststoffgehalt - (1 bis 15) : (1 bis 20) : (65 bis 98) Gewichtsteilen unter der Voraussetzung betragen, daß sich die Mengenangaben zu 100 Gew.-% ergänzen. In einer bevorzugten Ausführungsform der Erfindung, setzen sich die kosmetischen Zubereitungen wie folgt zusammen:
(a) 0,1 bis 5, vorzugsweise 0,5 bis 2 Gew.-% Dialkylether der Formel **(I)**,
(b) 0,1 bis 10, vorzugsweise 1 bis 5 Gew.-% Siliconverbindungen und
(c) 1 bis 50, vorzugsweise 5 bis 25 Gew.-% Emulgatoren,
mit der Maßgabe, daß sich die Mengenangaben mit Wasser und weiteren üblichen Hilfs- und Zusatzstoffen zu 100 Gew.-% ergänzen.

### Gewerbliche Anwendbarkeit

Die erfindungsgemäßen perlglänzenden, siliconhaltigen Zubereitungen zeichnen sich durch hohe Lagerstabilität und brillanten Perlglanz aus. Nachdem die Kombination aus ausgewählten Perlglanzmitteln und ausgewählten Emulgatoren für die Stabilität der Zubereitungen kritisch ist, betrifft ein weiterer Gegenstand der Erfindung die Verwendung von Mischungen, enthaltend
(a) Dialkylether der Formel **(I)**,
(b) Siliconverbindungen und
(c) Emulgatoren, ausgewählt aus der Gruppe, die gebildet wird von Alkyl- und/oder Alkenyloligoglykosiden und/oder Fettsäure-N-alkylpolyhydroxyalkylamiden, wie definiert im Patentanspruch 1.
zur Herstellung von perlglänzenden Siliconshampoos.

### Kosmetische Zubereitungen

Die erfindungsgemäßen Zubereitungen, wie beispielsweise Haarshampoos, Haarlotionen, Schaumbäder, Cremes, Lotionen oder Salben, können femer als weitere Hilfs- und Zusatzstoffe weitere milde Tenside, Ölkörper, Co-Emulgatoren, Überfettungsmittel, Stabilisatoren, Wachse, Konsistenzgeber, Verdickungsmittel, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Typische Beispiele für geeignete milde, d.h. besonders hautverträgliche **Tenside** sind Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fett-alkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Ester der Benzoesäure mit C₆-C₂₂-Kohlenstoffatomen, Guerbetcarbonate und/oder aliphatische bzw. naphthenische Kohlenwasserstoffe in Betracht.

Als **Co-Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
(4) Anlagerungsprodukte von 15 bis 60 Mol Ethylenoxid an Ricinusöl und/oder gehärtetes Ricinusöl;
(5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(6) Anlagerungsprodukte von 2 bis 15 Mol Ethylenoxid an Ricinusöl und/oder gehärtetes Ricinusöl;
(7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(8) Trialkylphosphate sowie Mono-, Di- und/oder Tri-PEG-alkylphosphate;
(9) Wollwachsalkohole;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt. Neben den nichtionsichen kommen auch kationische Emulgatoren in Betracht, wobei solche vom Typ der Esterquats, vorzugsweise methyl-quatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethytcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als weitere **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminiumund/oder Zink-stearat eingesetzt werden. Unter **biogenen Wirkstoffen** sind beispielsweise Tocopherol, Tocopherol-acetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind bei-spielsweise Chitosan, mikrokristallines Chitosan, quatemiertes Chitosan, Polyvinylpyrrolidon, Vinylpyrrolidon-Vinylacetat-Copolymerisate, Polymere der Acrylsäure-reihe, quatemäre Cellulose-Derivate, Kollagen, Hyaluronsäure bzw. deren Salze und ähnliche Ver-bindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei ei-nen mittleren Durchmesser von weniger als 100 nm, vorzugsweise zwischen 5 und 50 nm und ins-besondere zwischen 15 und 30 nm aufweisen. Sie können eine sphärische Form aufweisen, es können jedoch auch.solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt wer-den, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversiönstemperatur-Methode.

### Beispiele

Die Testformulierungen wurden 14 Tage bei 20°C gelagert und anschließend bezüglich Lagerstabilität und Perlglanz subjektiv beurteilt. Es bedeuten für die Lagerstabilität : (+++) unverändert, (++) kaum merkliche Eintrübung, (+) deutliche Eintrübung und (-) Aufrahmung der Siliconkomponente. Es bedeuten für den Perlglanz : (+++) brillant, (++) mäßig brillant und (+) eher stumpf. Die avivierenden Wirkung wurde über die Naßkämmbarkeit von Haarsträhnen bestimmt. Hierzu wurde die Kämmarbeit in mV für eine unbehandelte und eine gleichartige mit der Testlösung behandelte Haarsträhne gemessen. Angegeben ist die Differenz zwischen diesen beiden Wert als Mittelwert aus drei Bestimmungen. Je größer die ausgewiesene Differenz ist, um so deutlicher wird die Kämmarbeit vermindert und damit die Kämmbarkeit verbessert. Die hautkosmetische Verträglichkeit wurde über den Reizsummenscore gegenüber einer Standardrezeptur bestimmt. Die Ergebnisse sind in Tabelle 1 zusammengefaßt. Die Rezepturen 1 und 2 sind erfindungsgemäß, die Formulierungen V1 und V2 dienen zum Vergleich.

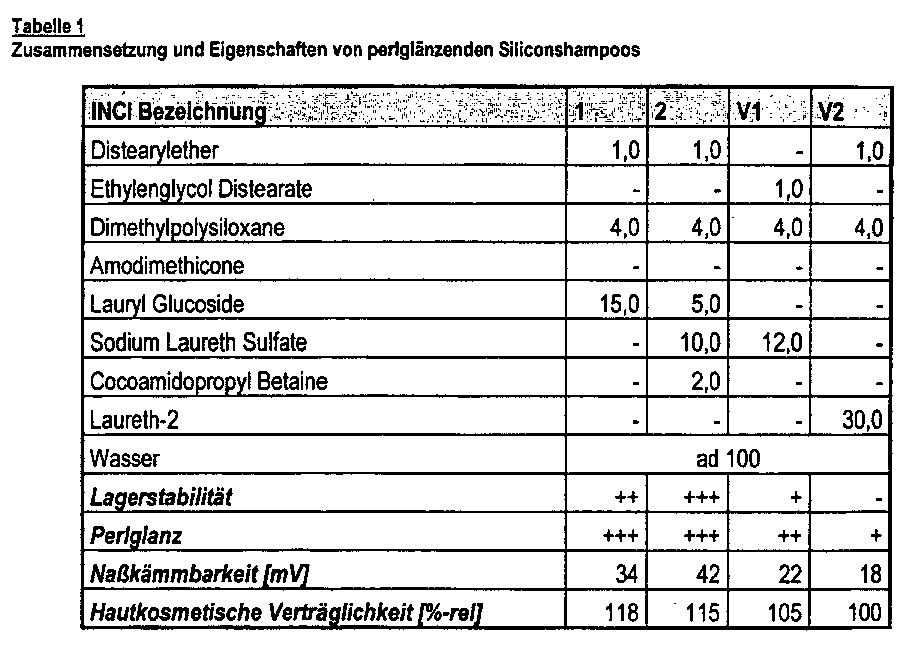

## Patentansprüche

1. Kosmetische Zubereitungen, enthaltend
(a) Dialkylether der Formel **(I),**
**R**^{**1**}**-O-R**^{**2**} **(I)**
in der R¹ und R² unabhängig voneinander für lineare oder verzweigte Alkyl- und/oder Alkenylreste mit 12 bis 22 Kohlenstoffatomen stehen,
(b) Siliconverbindungen und
(c) Emulgatoren ausgewählt aus der Gruppe, die gebildet wird von Alkyl- und/oder Alkenyloligoglykosiden der Formel **(II)**
**R**^{**3**}**O-[G]**_{**p**} **(II)**
in der R³ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht
und/oder Fettsäure-N-alkylpolyhydroxyalkylamiden.

2. Zubereitungen nach Anspruch 1, **dadurch gekennzeichnet, daß** sie als Komponente (a) Distearylether enthalten.

3. Zubereitungen nach den Ansprüchen 1 und 2, **dadurch gekennzeichnet, daß** sie als Komponente (b) Siliconverbindungen enthalten, die ausgewählt sind aus der Gruppe, die gebildet wird von Dimethylpolysiloxanen, Methylphenylpolysiloxanen, cyclischen Silicone sowie amino-, fettsäure-, alkohol-, polyether-, epoxy-, fluor- und/oder alkylmodifizierten Siliconen.

4. Zubereitungen nach den Ansprüchen 1 bis 3, **dadurch gekennzeichnet, daß** sie als Komponente (c) Fettsäure-N-alkylpolyhydroxyalkylamide der Formel **(III)** enthalten. 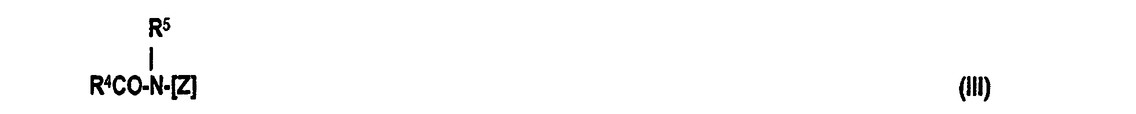 in der R⁴CO für einen aliphatischen Acylrest mit 6 bis 22 Kohlenstoffatomen, R⁵ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

5. Zubereitungen nach den Ansprüchen 1 bis 4, **dadurch gekennzeichnet, daß** sie
(a) 0,1 bis 5, vorzugsweise 0,5 bis 2 Gew.-% Dialkylether der Formel **(I)**,
(b) 0,1 bis 10, vorzugsweise 1 bis 5 Gew.-% Siliconverbindungen und
(c) 1 bis 50, vorzugsweise 5 bis 25 Gew.-% Emulgatoren,
mit der Maßgabe enthalten, daß sich die Mengenangaben mit Wasser und weiteren üblichen Hilfs- und Zusatzstoffen zu 100 Gew.-% ergänzen.

6. Verwendung von Mischungen nach Anspruch 1 zur Herstellung von perlglänzenden Siliconshampoos.

## Claims

1. Cosmetic preparations containing
(a) dialkyl ethers corresponding to formula **(I):**
**R**^{**1**}**-O-R**^{**2**} **(I)**
in which R¹ and R² independently of one another represent linear or branched alkyl and/or alkenyl groups containing 12 to 22 carbon atoms,
(b) silicone compounds and
(c) emulsifiers selected from the group consisting of alkyl and/or alkenyl oligoglycosides corresponding to formula **(II):**
**R**^{**3**}**O-[G]**_{**p**} **(II)**
where R³ is an alkyl and/or alkenyl group containing 4 to 22 carbon atoms, G is a sugar unit containing 5 or 6 carbon atoms and p is a number of 1 to 10,
and/or fatty acid-N-alkyl polyhydroxyalkylamides.

2. Preparations as claimed in claim 1, **characterized in that** they contain distearyl ether as component (a).

3. Preparations as claimed in claims 1 and 2, **characterized in that** they contain as component (b) silicone compounds selected from the group consisting of dimethyl polysiloxanes, methyl phenyl polysiloxanes, cyclic silicones and amino-, fatty acid-, alcohol-, polyether-, epoxy-, fluorine- and/or alkyl-modified silicones.

4. Preparations as claimed in claims 1 to 3, **characterized in that** they contain as component (c) fatty acid-N-alkyl polyhydroxyalkyl amides corresponding to formula **(III):** 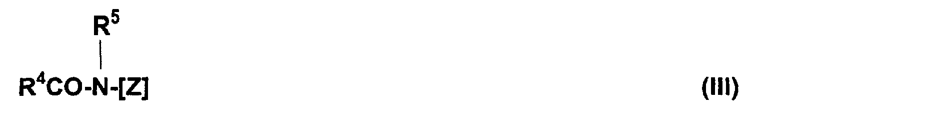 where R⁴CO is an aliphatic acyl group containing 6 to 22 carbon atoms, R⁵ is an alkyl or hydroxyalkyl group containing 1 to 4 carbon atoms and [Z] is a linear or branched polyhydroxyalkyl group containing 3 to 12 carbon atoms and 3 to 10 hydroxyl groups.

5. Preparations as claimed in claims 1 to 4, **characterized in that** they contain
(a) 0.1 to 5 and preferably 0.5 to 2% by weight dialkyl ethers corresponding to formula **(I)**,
(b) 0.1 to 10 and preferably 1 to 5% by weight silicone compounds and
(c) 1 to 50 and preferably 5 to 25% by weight emulsifiers,
with the proviso that the quantities add up to 100% by weight with water and other typical auxiliaries and additives.

6. The use of the mixtures claimed in claim 1 for the production of pearlescent silicone shampoos.

## Revendications

1. Préparations cosmétiques contenant :
a) des éthers dialkyliques de formule (I) :
R¹-0-R² (I)
dans laquelle R¹ et R² indépendamment l'une de l'autre représentent des restes alkyle et/ou alkényle, linéaires ou ramifiés, ayant de 12 à 22 atomes de carbone,
b) des composés de silicone et,
c) des agents émulsionnants choisis dans le groupe formé des alkyl- et/ou d'alkényloligoglycosides de formule (II) :
R³O-[G]ₚ (II)
dans laquelle R³ représente un reste alkyle et/ou alkényle ayant de 4 à 22 atomes de carbone, G représente un reste de sucre ayant 5 ou 6 atomes de carbones et p représente des nombres allant de 1 à 10 et/ou des N-alkylpolyhydroxyalkylamides d'acide gras.

2. Préparations selon la revendication 1,
**caractérisées en ce qu'**
elles renferment comme composant a) l'éther distéarylique.

3. Préparations selon les revendications let 2,
**caractérisées en ce qu'**
elles renferment comme composant (b) des composés de silicone qui sont choisies dans le groupe formé des diméthylpolysiloxanes, des méthylphénylpolysiloxanes, des silicones cycliques ainsi que des silicones modifiées par un amino, un acide gras, un alcool, un polyéther, un époxy, un fluor et/ou un alkyle.

4. Préparations selon quelconque des revendications 1 à 3,
**caractérisées en ce qu'**
elles renferment comme composant c) des N-alkylpolyhydroxyalkylamides d'acide gras de formule (III) 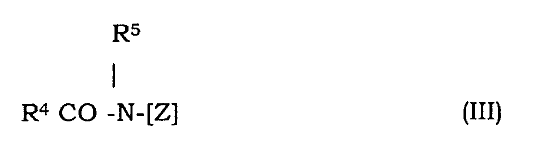 dans laquelle R⁴CO représente un reste acyle aliphatique ayant de 6 à 22 atomes de carbone, R⁵ représente un reste alkyle ou hydroxyalkyle ayant de 1 à 4 atomes de carbone, et [Z] représente un reste un reste polyhydroalkyle linéaire ou ramifié ayant de 3 à 12 atomes de carbone et de 3 à 10 groupes hydroxyle.

5. Préparations selon l'une quelconque des revendications 1 à 4,
**caractérisées en ce qu'**
elles renferment
(a) de 0,1 à 5, de préférence de 0,5 à 2 % en poids d'éther dialkylique de formule (I)
(b) de 0,1 à 10, de préférence de 1 à 5 % en poids de composés des silicones et,
(c) de 1 à 50, de préférence de 5 à 25 % d'agent émulsionnant
avec la précision que les données en quantités se complètent avec l'eau et d'autres adjuvants et additifs usuels à 100 % en poids.

6. Utilisation de mélanges de préparations selon la revendication 1, en vue de la préparation de shampooings aux silicones à éclat nacré.
